# EUROPEAN PATENT APPLICATION

(11) **EP 4 516 903 A1**
(43) Date of publication of application: **05.03.2025**
(21) Application number: 23806965.2
(22) Date of filing: 17.05.2023
(51) Int. Cl.: C12N 15/11, C12N 15/10, C40B 50/06

(54) **SGRNA SEQUENCING LINKER AND USE THEREOF**

(30) Priority: 17.05.2022 CN 202210541595
(71) Applicant: Nanjing GenScript Biotech Co., Ltd., Nanjing, Jiangsu 211122 (CN)
(72) Inventor: CHEN, Luyao, Nanjing, Jiangsu 211122 (CN); MAO, Yibo, Nanjing, Jiangsu 211122 (CN); SHENG, Xia, Nanjing, Jiangsu 211122 (CN); FAN, Long, Nanjing, Jiangsu 211122 (CN); LI, Hong, Nanjing, Jiangsu 211122 (CN)
(74) Representative: Laufhütte, Dieter
(86) International application number: PCT/CN2023/094691
(87) International publication number: WO 2023/222014

(57) **Abstract**

The present invention relates to the technical field of molecular biology, and in particular, to a sgRNA sequencing linker and use thereof. The sgRNA sequencing 3' linker sequentially comprises the following sections from a 5'-end to a 3'-end: a first non-random section, a first random section, a second non-random section, a loop-forming DNA section, and a third non-random section, wherein the first non-random section is used for being linked to the 3'-end of the sgRNA; the first random section comprises 3 to 12 basic groups; the second non-random section is reversely complementary to the third non-random section so as to form a neck ring structure in conjuncture with the loop-forming DNA section; the third non-random section is used as a primer for sgRNA reverse transcription and replication; the loop-forming DNA section is composed of a first loop-forming section and a second loop-forming section from the 5'-end to the 3'-end; the third non-random section and the second loop-forming section can be combined with the first sequencing linker primer sequence in a complementary pairing mode.

## Description

### Cross Reference to Related Application

The present application claims priority to Chinese Patent Application No. 2022105415956, filed with the China National Intellectual Property Administration on May 17, 2022 and entitled "SGRNA SEQUENCING LINKER AND USE THEREOF", which is incorporated herein by reference in its entirety.

### Technical Field

The present invention relates to the technical field of molecular biology, and in particular, to a sgRNA sequencing linker and the use thereof.

### Background Art

Next generation sequencing, also referred to as high-throughput sequencing, can sequence hundreds of thousands to millions of DNA (deoxyribonucleic acid) molecules in parallel at one time. The technology has been widely used in many fields such as medical treatment, new drug research and development, livestock breeding, forensic evidence identification, customs quarantine and identification, and molecular biology research due to the characteristics of high sequencing throughput, short sequencing time, low sequencing cost, high sequencing accuracy, etc.

Although next generation sequencing has been widely used, there are still technical difficulties in sequencing sgRNA. sgRNA (single guide RNA) is a key component of CRISPR/Cas gene editing technology, which guides Cas protein to cleave genome and is a main factor that determines the gene editing efficiency. When gene editing is performed with a CRISPR/Cas technique using an artificially synthesized sgRNA, the higher the sequence accuracy of the sgRNA, the higher the accuracy thereof in guiding Cas protein to bind and cleave a target DNA sequence. Therefore, the sgRNA sequencing technique can accurately detect the single-stranded oligonucleotide sequence of sgRNA. The sgRNA with high sequence accuracy can improve the gene editing efficiency of the CRISPR/Cas technology. In addition, during the synthesis of the oligonucleotide strand of sgRNA, chemical modifications are performed at the 5'-end and the 3'-end to improve the storage stability of RNA samples. However, these chemical modifications often increase the difficulty of sgRNA sequencing library construction, reduce the yield of sequencing libraries, and even lead to the failure of sequencing library construction. Moreover, the sgRNA sequence is short, and due to the PCR amplification bias of the library and the linking bias of the linker, etc., the nucleic acid to be sequenced will not be amplified in the same proportion, and different types of small-fragment RNAs are detected too many or too few times, resulting in differences between the sequencing result and the original abundance in the sample.

In order to solve the problems of low library yield, difficulty in library construction, etc. in the existing sgRNA sequencing library construction, there is a need to develop a new library construction method.

### Summary of the Invention

A first aspect of the present invention relates to a sgRNA sequencing 3' linker sequentially comprising the following sections from a 5'-end to a 3'-end: a first non-random section, a first random section, a second non-random section, a loop-forming DNA section, and a third non-random section,
wherein the first non-random section is used for being linked to the 3'-end of the sgRNA;
the first random section comprises 3, 4, 5, 6, 7, 8, 9, 10, 11 or 12 basic groups;
the second non-random section is reversely complementary to the third non-random section so as to form a neck ring structure in conjuncture with the loop-forming DNA section; the third non-random section is used as a primer for sgRNA reverse transcription and replication;
the loop-forming DNA section is composed of a first loop-forming section and a second loop-forming section from the 5'-end to the 3'-end;
the third non-random section and the second loop-forming section can be combined with the first sequencing linker primer sequence in a complementary pairing mode.

A second aspect of the present invention relates to a linker set comprising the 3' linker as described above and a 5' linker used for being linked to the 5'-end of the sgRNA,
wherein the 5' linker is composed of ribonucleotides, and sequentially comprises a second sequencing linker primer-binding section, a second random section, and a fourth non-random section from a 5'-end to a 3'-end; the second random section comprises 3, 4, 5, 6, 7, 8, 9, 10, 11 or 12 basic groups; the fourth non-random section is reversely complementary to the first non-random section.

A third aspect of the present invention relates to a kit comprising the linker set as described above.

A fourth aspect of the present invention relates to a method for constructing a sgRNA sequencing library, which method uses the linker set as described above and comprises the following steps:
a) subjecting sgRNA to a linking reaction with the 3' linker under suitable conditions;
b) adding the 5' linker to the product obtained in the reaction of step a), and performing annealing and blocking under suitable conditions, such that the fourth non-random section of the 5' linker hybridizes with the first non-random section of the 3' linker to form a double strand;
c) subjecting the product obtained in step b) to a linking reaction under suitable conditions, such that the 5' linker is linked to the sgRNA;
d) subjecting the product obtained in step c) to a reverse transcription reaction under suitable conditions to obtain cDNA;
e) adding first and second sequencing linker primers with indexes to both ends of the cDNA and enriching the library.

A fifth aspect of the present invention relates to a sgRNA sequencing method, which comprises:
1) constructing a sgRNA sequencing library using the method as described above;
2) sequencing the sgRNA sequencing library obtained in step 1); wherein preferably sequencing is performed using an Illumina sequencing platform.

A sixth aspect of the present invention relates to the use of the 3' linker as described above or the linker set as described above in the construction of a sgRNA library.

A seventh aspect of the present invention relates to a constructed sgRNA sequencing library, wherein the sgRNA sequencing library is constructed by the method comprising:
a) subjecting sgRNA to a linking reaction with the 3' linker under suitable conditions;
b) adding the 5' linker to the product obtained in the reaction of step a), and performing annealing and blocking under suitable conditions, such that the fourth non-random section of the 5' linker hybridizes with the first non-random section of the 3' linker to form a double strand;
c) subjecting the product obtained in step b) to a linking reaction under suitable conditions, such that the 5' linker is linked to the sgRNA;
d) subjecting the product obtained in step c) to a reverse transcription reaction under suitable conditions to obtain cDNA;
e) adding first and second sequencing linker primers with indexes to both ends of the cDNA and enriching the library.

An independently designed and developed linker with random sequence basic groups and a fixed sequence used in the present invention can be used as a molecule identifier to effectively reduce the background noise introduced during library construction, PCR amplification, and sequencing, and can effectively reduce the linking bias of the linker for substrate RNAs of different structural types, and eliminate the interference of the PCR amplification bias on the quantification of RNA molecules, thereby truly reflecting the RNA abundance and target sequence information in the sample.

An independently developed semi-circular linker with a molecule identifier used in the present invention has higher linking efficiency than traditional single-strand linker. Moreover, in the reverse transcription, the semi-circular linker can be used as a reverse transcription primer for direct reverse transcription without the addition of a reverse transcription primer during reverse transcription, thereby reducing the cost and the short fragment contamination of the reverse transcription primer. Since the sequences of universal primers can be adjusted according to a sequencing platform, the semi-circular linker has a wider range of applications, the addition of special sequencing primers during sequencing is not required, and non-specific amplification products such as linker dimers can be effectively reduced. The linkers of the present invention, due to the advantages of linking efficiency, low dimer contamination, etc., successfully realize the construction of a library of sgRNAs containing a modification, with a high construction success rate and low cost.

### Brief Description of the Drawings

To describe the technical solutions in detailed description of embodiments of the present invention or in the prior art more clearly, the accompanying drawings required in the description of detailed description of embodiments or the prior art are briefly described below. It is clear that the accompanying drawings in the following description illustrate merely some embodiments of the present invention, and those of ordinary skill in the art may further derive other drawings from these accompanying drawings without creative efforts.

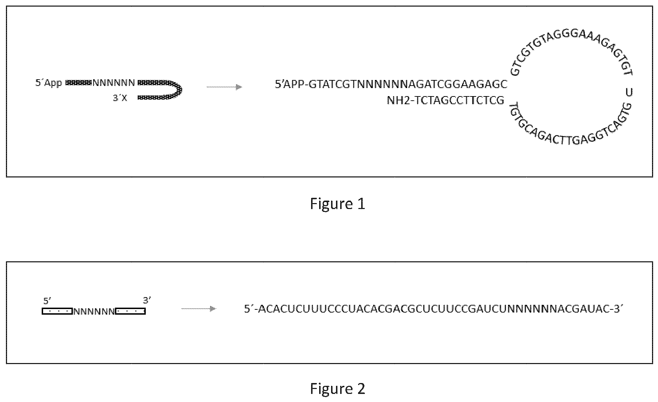

FIG. 1 is a schematic structural diagram of a UMA3 linker provided in the examples of the present invention;
FIG. 2 is a schematic structural diagram of a UMA5 linker provided in the examples of the present invention;

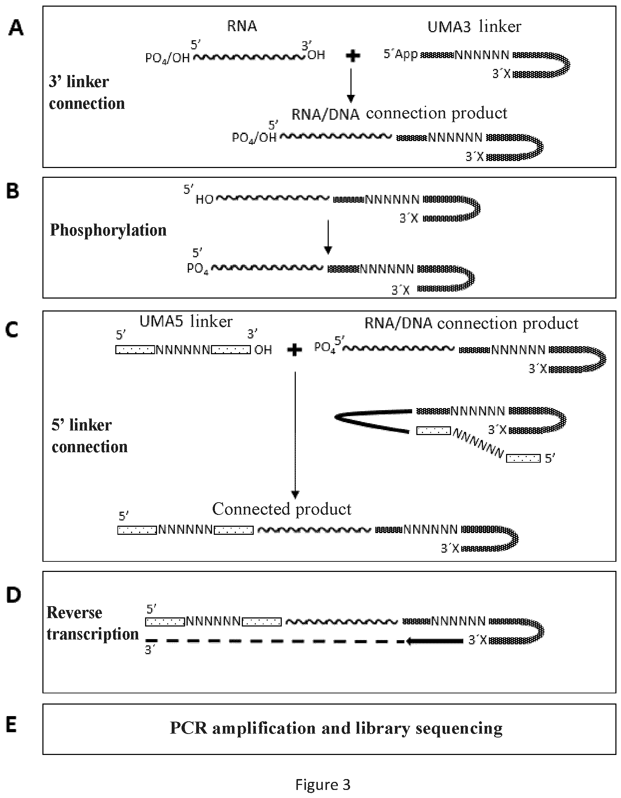

FIG. 3 is a schematic flow diagram of the construction of a sgRNA sequencing library provided in the examples of the present invention;
FIG. 4 is a graph showing fragment length results analyzed by Agilent 2100 Bioanalyser instrument for a sequencing library of sgRNA samples having a length of 100 nt constructed according to experimental procedures in examples of the present invention; peak I is a linker dimer; peak II is the library of interest;

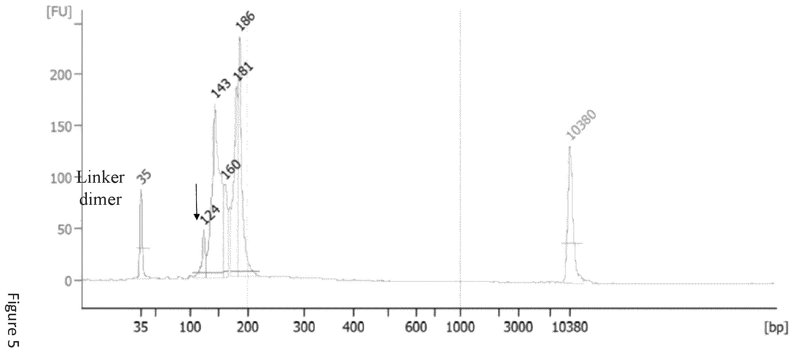

FIG. 5 is a graph showing results of sequencing library of sgRNA samples having a length of 100 nt constructed using commercially available library construction kit in some examples;
FIG. 6 is a graph showing results of sequencing library of sgRNA samples having a length of 100 nt constructed in some examples of the present invention;

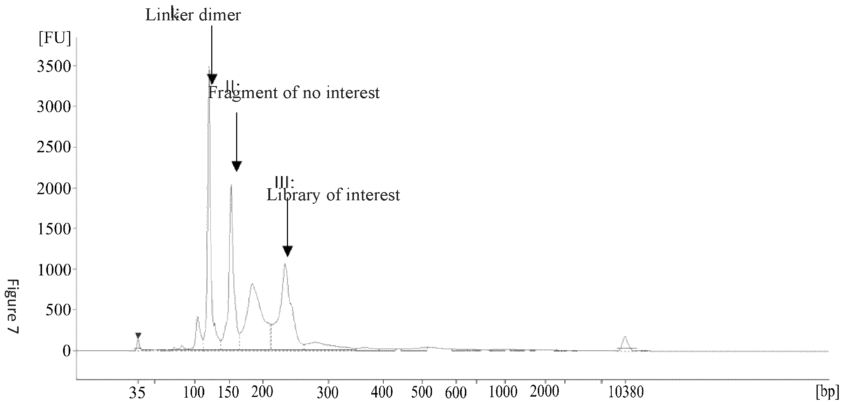

FIG. 7 is a graph showing results of sequencing library of sgRNA samples having a length of 100 nt constructed using commercially available library construction kit in some examples.

### Detailed Description of Embodiments

Reference now will be made in detail to the embodiments of the present invention, one or more examples of which are set forth below. Each example is provided as an explanation rather than limiting the present invention. Indeed, it would have been obvious to a person skilled in the art that various modifications and variations may be made to the present invention without departing from the scopes or spirits of the present invention. For instance, features illustrated or described as part of one embodiment, can be used on another embodiment to yield a still further embodiment.

Unless otherwise stated, all terms (including technical and scientific terms) used to disclose the present invention have the same meaning as commonly understood by those of ordinary skill in the art to which the present invention belongs. With further guidance, ensuing definitions are used to better understand the teachings of the present invention. Herein, the terms used in the description of the present invention are merely for the purpose of describing specific examples, but are not intended to limit the present invention.

The terms "and/or" and "or/and" used herein are selected to encompass any one of two or more associated items listed therein, as well as any and all combinations of the associated items listed therein, wherein the combinations include combinations of any two of the associated items listed therein, any more of the associated items listed therein, or all of the associated items listed therein. It should be noted that when at least three items are connected by a combination of at least two conjunctions selected from "and/or" and "or/and", it should be understood that in the present application, the technical solutions definitely include the technical solution in which items are all connected by "logic AND" and also definitely include the technical solutions in which items are all connected by "logic OR". For example, "A and/or B" includes the three parallel solutions of A, B and A+B. As another example, the technical solution of "A, and/or, B, and/or, C, and/or, D" includes any one of A, B, C, and D (i.e., the technical solutions in which items are all connected by "logic OR"), also includes any and all combinations of A, B, C, and D, that is, combinations of any two or any three of A, B, C, and D, and further includes the combination of all the four items A, B, C, and D (i.e., the technical solution in which items are all connected by "logic AND").

The terms "contain", "comprise", and "include" used herein are synonymous, inclusive or open-ended, and do not exclude additional, unrecited members, elements, or method steps.

Numerical ranges expressed by endpoints in the present invention include all numbers and fractions included within the range, as well as the recited endpoints.

The present invention relates to a concentration value, and the fluctuations of the value are within a certain range. For example, it can fluctuate within the corresponding precision range. For example, with regard to the value 2%, fluctuations within the range of ±0.1% may be permitted. For larger values or values that do not require way too fine control, the meaning is also allowed to include larger fluctuations. For example, with regard to the value 100 mM, fluctuations within the range of ±1%, ±2%, ±5%, etc. may be permitted.

In the present invention, expressions involving terms such as "a plurality of" and "multiple" refer to a quantity greater than or equal to 2, unless otherwise specified.

In the present invention, the technical features described in an open-ended manner include both a closed technical solution consisting of the listed features and an open-ended technical solution comprising the listed features.

In the present invention, the expressions "preferably", "preferentially", "more preferably", and "appropriately" are solely used for describing better embodiments or examples, and it should be understood that the scope of the present invention is not intended to be limited. In the present invention, the expressions "optionally", "optional" and "alternative" mean that the subject modified by the expressions are dispensable, that is, the expressions mean that the parallel technical solutions "with" or "without" the subject modified by the expressions can both be selected. If the expression "alternative" appears multiple times in a technical solution, unless otherwise specified and without any contradictions or mutually restrictive relationships, the expression "alternative" is independent in each occurrence.

In the present invention, the term "nucleic acid", "nucleotide" or "polynucleotide" refers to deoxyribonucleic acid (DNA), ribonucleic acid (RNA) and a polymer thereof in a single-, double- or multi-stranded form. The term includes, but is not limited to, single-, double-, or multi-stranded DNA or RNA, genomic DNA, cDNA, DNA-RNA hybrids, or a polymer comprising purine and/or pyrimidine basic groups or other natural, chemically modified, biochemically modified, unnatural, synthetic, or derivatized nucleotide basic groups. In some embodiments, the nucleic acid may comprise a mixture of DNA, RNA, and an analog thereof. Unless specifically defined, the term encompasses nucleic acids that contain known analogs of natural nucleotides, have binding properties similar to reference nucleic acids, and are metabolized in a manner similar to naturally occurring nucleotides. Unless otherwise indicated, a specific nucleic acid sequence also implicitly encompasses conservatively modified variants thereof (e.g., degenerate codon substitutions), alleles, orthologs, single nucleotide polymorphisms (SNPs), and complementary sequences, as well as explicitly indicated sequences. Specifically, the degenerate codon substitutions may be achieved by: generating a sequence in which the third positions of one or more selected (or all) codons are substituted with mixed basic groups and/or deoxyinosine residues (Batzer et al., Nucleic Acid Res. 19: 5081 (1991); Ohtsuka et al., J. Biol. Chem. 260: 2605-2608 (1985); and Rossolini et al., Mol. Cell. Probes 8: 91-98 (1994)). The term "nucleic acid" may be used interchangeably with a gene, cDNA and mRNA encoded by a gene.

In the present invention, the term "sgRNA", also referred to as single guide RNA, guide RNA, or gRNA, refers to an RNA molecule that can form a complex with a Cas protein in a CRISPR system and can target the complex to a target sequence due to some complementarity with the target sequence. For example, in a Cas9-based gene editing system, gRNA is generally composed of crRNA and tracrRNA molecules that are partially complementary to form a complex, wherein the crRNA comprises a sequence that has sufficient complementarity to a target sequence to hybridize with the target sequence and guides a CRISPR complex (Cas9 + crRNA + tracrRNA) to be specifically combined with the target sequence. It is known in the art that a sgRNA that has features of both crRNA and tracrRNA can be designed. However, in a Cpf1-based genome editing system, a sgRNA is generally composed only of a mature crRNA molecule, wherein the crRNA comprises a sequence that has sufficient identity to a target sequence to hybridize with a complementary sequence of the target sequence and guides a complex (Cpf1 + crRNA) to be specifically combined with the target sequence. Designing a suitable sgRNA sequence on the basis of the used CRISPR/Cas system and the target sequence to be edited is within the competence of a person skilled in the art. The sgRNA of the present invention may comprise other structures or modifications known in the art that are used for improving its properties. For example, the sgRNA may comprise an additional MS2 hairpin aptamer sequence (e.g., inserted into the stem-loop structure), such that it may be combined with an MS2 protein to provide additional functions for the gene editing system; or for example, the sgRNA may comprise one or more modified nucleotides, such as comprising a modification in a ribose group, a phosphate group, a nucleobase, or a combination thereof. The modification in a ribose group may be a modification at the 2' position of the ribose group. In some cases, the modification at the 2' position of the ribose group is selected from the group consisting of: 2'-O-methyl, 2'-fluoro, 2'-deoxy, 2'-O-methyl 3' phosphorothioate (MS), or 2'-O-methyl 3' thioPACE (MSP). Studies have shown that the modification can enhance the stability of sgRNA as well as crRNA and tracRNA (Hendel et al., 2015; and Rahdar et al., 2015).

In the present invention, the term "random section" refers to a region of a sequence in which any nucleotide or basic group can occur. For example, in the chemical synthesis of an oligonucleotide, the incorporation of any nucleotide at any position can be achieved by introducing a mixture of nucleotides (dA, dG, dC and dT commonly used for DNA oligonucleotides, and dA, dG, dC and dU commonly used for RNA oligonucleotides) in a chemical reaction of extending the oligonucleotide strand.

In the present invention, the term "non-random section" refers to a specific position in an oligonucleotide at which at least one specific nucleotide or basic group is incorporated. For example, in a chemical reaction of extending an oligonucleotide strand, one or more nucleotides can be introduced into a specific position to synthesize a specific nucleotide sequence.

The present invention relates to a sgRNA sequencing 3' linker sequentially comprising the following sections from a 5'-end to a 3'-end: a first non-random section, a first random section, a second non-random section, a loop-forming DNA section, and a third non-random section,
wherein the first non-random section is used for being linked to the 3'-end of the sgRNA;
the first random section comprises 3, 4, 5, 6, 7, 8, 9, 10, 11 or 12 basic groups;
the second non-random section is reversely complementary to the third non-random section so as to form a neck ring structure in conjuncture with the loop-forming DNA section; the third non-random section is used as a primer for sgRNA reverse transcription and replication;
the loop-forming DNA section is composed of a first loop-forming section and a second loop-forming section from the 5'-end to the 3'-end;
the third non-random section and the second loop-forming section can be combined with the first sequencing linker primer sequence in a complementary pairing mode.

The 3' linker is also referred to as a UMA3 linker in the present invention because it is linked to the 3'-end of the sgRNA.

A random sequence can effectively reduce the linking bias of a linker for substrate RNAs of different structural types, and can also be used as a unique molecule identifier (UMI) to effectively reduce the background noise introduced during library construction, PCR amplification, and sequencing, and eliminate the interference of PCR amplification bias on the quantification of RNA molecules, thereby truly reflecting the RNA abundance and target sequence information in the sample.

The loop-forming DNA section is not complementary to other sequences, does not comprise complementary sequences inside, and forms a loop in the UMA3 linker structure, which is conducive to the stability of the linker sequence. By the clever design, the third non-random section of the UMA3 linker is reversely complementary to the second non-random section thereof and can be used as a primer for reverse transcription, which simplifies the experimental operations. Moreover, the loop-forming DNA section can be combined with the first sequencing linker primer in a complementary pairing mode, which further simplifies the overall experimental process.

The 3' linker can comprise one or more ribonucleotides, but is preferably composed of deoxyribonucleotides.

In some embodiments, the first non-random section comprises 5, 6, 7, 8, 9, 10, 11 or 12 basic groups. The first non-random section consists of 5 to 12 A/T/C/G basic groups freely arranged and combined. For example, when the first non-random section is 5 nt in length, there are a total of 4⁵ = 1024 types of the first non-random section. In some specific embodiments, the first non-random section is 7 nt in length. In some specific embodiments, the sequence of the first non-random section is GTATCGT.

In some embodiments, the sequence of the third non-random section can be combined with the first sequencing linker primer sequence in a complementary pairing mode to further increase the utilization rate thereof.

In some embodiments, the third non-random section comprises 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 basic groups. In some specific embodiments, the third non-random section comprises a sequence set forth in SEQ ID NO: 1.

In some embodiments, the second loop-forming section comprises 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33 basic groups, and preferably comprises 10 to 30 basic groups. In some specific embodiments, the second loop-forming section comprises a sequence set forth in SEQ ID NO: 3.

In some embodiments, the third non-random section and the second loop-forming section comprise a total of 34 basic groups.

In the present invention, the random sequence is generally expressed in the form of "NNNNN" (when it is 5 nt), where N represents any one of A/T/C/G/U basic groups. The length of the random sequence is not specified, as long as the number of combinations thereof is sufficient to distinguish all the molecules comprised in the same sample. In order to achieve the purpose of each molecule in a sample being labeled with different types (i.e., different combinations of basic group sequences) of UMIs, it is generally required that the number of types of UMIs is much greater than the number of molecules. In some embodiments, in consideration of the cost, the random sequence comprises 3-12 basic groups, such as 3, 4, 5, 6, 7, 8, 9, 10, 11 or 12 basic groups. In some specific embodiments, the random sequence is 6 nt in length.

In some embodiments, the sequence of the first loop-forming section comprises a sequence set forth in SEQ ID NO: 2.

In some embodiments, furthermore, a structure that can be cleaved by a protease is comprised between the first loop-forming section and the second loop-forming section. In the present invention, the expression "protease cleavage" refers to that an exposed nucleic acid for being combined with a primer can be formed after treatment with a protease, and thus it may be a complete separation of a nucleic acid strand, or may be in other forms, such as an abasic site. For the structure that can be cleaved by a protease, it is preferably cleaved by the incorporation of one or more deoxyuridines (dUs); and the enzyme used for cleavage may be an enzyme having uracil-DNA glycosylase activity and AP-endonuclease activity to form an abasic site. The expression "protease cleavage" may also further include cleaving a polynucleotide strand comprising an abasic site at the abasic site by endonuclease (such as EndolV endonuclease, AP lyase, FPG glycosylase/AP lyase, EndoVlll glycosylase/AP lyase), heat or alkaline treatment, as long as the polynucleotide strand can be cleaved.

In some embodiments, the linker further comprises a nucleotide modification at the 5'-end and/or 3'-end.

In some embodiments, the linker comprises an adenylation modification at the 5'-end.

In some embodiments, the linker comprises an amino modification at the 3'-end.

The present invention further relates to a linker set comprising the 3' linker as described above and a 5' linker used for being linked to the 5'-end of the sgRNA,
wherein the 5' linker is composed of ribonucleotides, and sequentially comprises a second sequencing linker primer-binding section, a second random section, and a fourth non-random section from a 5'-end to a 3'-end; the second random section comprises 3, 4, 5, 6, 7, 8, 9, 10, 11 or 12 basic groups; the fourth non-random section is reversely complementary to the first non-random section.

The 5' linker is also referred to as a UMA5 linker in the present invention because it is linked to the 5'-end of the sgRNA.

The fourth non-random section at the 3'-end of the 5' linker is reversely complementary to the first non-random section, and can block the UMA3 linker under annealing conditions, thereby improving the linking efficiency.

The UMA5 linker sequence comprises a second sequencing linker primer-binding section, which can be combined with sequencing linker primer sequences with indexes during the first round of nucleic acid synthesis of a PCR enrichment library. The sequence of the sequencing linker primer-binding section can be designed by a person skilled in the art according to actual requirements. In some embodiments, the sequencing linker primer-binding section comprises 17 to 33 basic groups, e.g., 18, 19, 20, 21, 22, 23, 24, 25, 26, 27, 28, 29, 30, 31 or 32 basic groups. In some specific embodiments, the sequence of the second sequencing linker primer-binding section is ACACGACGCUCUUCCGAUCU (SEQ ID NO: 7), UACACGACGCUCUUCCGAUCU (SEQ ID NO: 8), or CCCUACACGACGCUCUUCCGAUCU (SEQ ID NO: 9). In some specific embodiments, the sequence of the second sequencing linker primer-binding section comprises 33 basic groups, and the basic group sequence is set forth in SEQ ID NO: 4.

The first and second sequencing linker primers in the present invention may be selected by a person skilled in the art as required. The sequence of the sequencing linker primer can be designed by a person skilled in the art as required, for example, by adding a suitable index for sample distinction to the sequence, etc. In some specific embodiments, the sequencing linker primer is preferably a known standard sequencing linker primer; more preferably, the first and second sequencing linker primers of the present invention are standard sequencing linker primers suitable for an Illumina sequencing platform, and comprise a sequencing primer-binding site for initiating sequencing; an I5 index can be added to the 3'-end of cDNA by PCR amplification, and then an I7 index can be added to the 3'-end of the amplified sequence after adding the I5 index by PCR amplification, in which the sequence positions of the indexes are fixed and the length can be 6 nt or 8 nt according to the setting of the sequencer.

In some specific embodiments, the first and second sequencing linker primers each comprise at least 17 basic groups, and in some specific embodiments, the first and second sequencing linker primers each comprise at least 33 basic group. In some specific embodiments, the first sequencing linker primer comprises a sequence set forth in SEQ ID NO: 6, and the second sequencing linker primer comprises a sequence set forth in SEQ ID NO: 5. In some embodiments, the first sequencing linker primer comprises a sequence set forth in SEQ ID NO: 14, and in some embodiments, the first sequencing linker primer comprises a sequence set forth in SEQ ID NO: 15.

The present invention further relates to a kit comprising the linker set as described above.

The term "kit" refers to any article of manufacture (e.g., package or container) that comprises at least one device. The article of manufacture can further comprise instructions for use, supplementary reagents, and/or components or assemblies used in the method or the step thereof described herein.

Preferably, the nucleic acid component and the protein component, e.g., the linker and the enzyme, in the kit are stored in the form of dry powder in the kit. Each component may also be provided in a lyophilized form, e.g., one or more so-called lyophilized beads. The lyophilized beads may generally be understood to refer to lyophilized substances that are compressed into spherical shapes after manufacture (after which the substances are usually present as a powder).

In some embodiments, the kit further comprises at least one of the following components:
·an RNA ligase;
·a linking buffer suitable for the RNA ligase;
·an enzyme or an enzyme composition having uracil-DNA glycosylase activity and AP-endonuclease activity;
·a reverse transcriptase;
·a reverse transcription reaction buffer;
·a DNA polymerase;
·a PCR amplification buffer for cDNA amplification;
·a T4 phosphokinase;
·a T4 phosphokinase reaction buffer;
·a first sequencing linker primer for being combined with the third non-random section and the second loop-forming section in a complementary pairing mode, and a second sequencing linker primer for being combined with the second sequencing linker primer-binding section in a complementary pairing mode;
·dNTPs; and
·water.

Some of the components are described in detail as follows:

### ·RNA ligase

In the present invention, "enzymes" (e.g., T4 RNA ligase, reverse transcriptase, and DNA polymerase) are to be understood in the maximum range known to a person skilled in the art. The range should include common enzymes and variants thereof having corresponding activity, and preferably, the RNA ligase is T4 RNA ligase. Taking T4 RNA ligase as an example, it should include a T4 RNA ligase, truncated, having RNA ligase activity (e.g., truncated KQ); the enzymes may also have common modifications or have tags (e.g., Arg-tag, His-tag, Strep-tag, Flag-tag, T7-tag, V5-peptide tag, GST-tag, and c-Myc-tag) linked thereto, as long as they have the desired activity. Further preferably, the T4 RNA ligase includes i) T4 RNA ligase 1, and/or ii) at least one of T4 RNA ligase 2, T4 RNA ligase 2, truncated, and T4 RNA ligase 2, truncated KQ.

### ·Linking buffer suitable for the RNA ligase

In the present invention, a buffer component/buffer system refers to an aqueous solution or composition that resists pH changes when an acid or base is added to the solution or composition. This resistance to pH changes is due to the buffering properties of such solutions. Buffers that can be used in the method of the present invention are preferably selected from phosphate buffer, phosphate buffered saline (PBS) buffer, 2-amino-2-hydroxymethyl-1,3-propanediol (Tris) buffer, Tris-NaCl buffer (TBS), and Tris/EDTA (TE).

Preferably, the linking buffer comprises a buffer component (such as Tris), Mg²⁺, and DTT; more preferably, the linking buffer is a buffer system comprising 7 mM to 13 mM Mg²⁺ and 0.7 mM to 1.3 mM DTT; more preferably, the linking buffer is a buffer system comprising 9 mM to 11 mM Mg²⁺ and 0.9 mM to 1.1 mM DTT; and preferably, the pH is 7 to 8.

### ·Enzyme or enzyme composition having uracil-DNA glycosylase activity and AP-endonuclease activity

The term "having uracil-DNA glycosylase activity" refers to the activity of recognizing uracil present in a single-stranded or double-stranded DNA and cleaving the N-glycosidic bond between the uracil basic group and deoxyribose, leaving an abasic site. Uracil-DNA glycosylases, abbreviated as "UDG" or "UNG" (EC 3.2.2.3), include mitochondrial UNG1, nuclear UNG2, SMUG1 (single-strand selective uracil-DNA glycosylase), TDG (TU mismatch DNA glycosylase), MBD4 (uracil-DNA glycosylase with a methyl binding region) and other prokaryotic and eukaryotic enzymes (see Krokan H. E. et al. "Uracil in DNA-occurrence, consequences and repair", Oncogene (2002) 21: 8935-9232).

In some preferred embodiments, the enzyme composition is a mixture of uracil-DNA glycosylase (UDG) and DNA glycosylase and lyase Endo VIII, e.g., "User enzyme".

### ·Reverse transcriptase

The reverse transcriptase can be selected from AMV reverse transcriptase, M-MuLV reverse transcriptase, etc. In some specific embodiments, the reverse transcriptase is M-MuLV reverse transcriptase. The reverse transcriptase is preferably thermostable. The reverse transcriptase may have no RNase H activity or have attenuated RNase H activity.

### ·DNA polymerase

The DNA polymerase can be selected from any one of Taq, Bst, Vent, Phi29, Pfu, Tru, Tth, Tl1, Tac, Tne, Tma, Tih, Tf1, Pwo, Kod, Sac, Sso, Poc, Pab, Mth, Pho, ES4 DNA polymerase, and Klenow fragment. The DNA polymerase is preferably a high-fidelity enzyme.

### ·Water

The water is preferably double distilled water or deionized water.

The above components are preferably free of nucleases (DNases and RNases), and if necessary, a nuclease inhibitor may be added.

Each component in the kit can be packaged independently or can be packaged in a mixture form of at least two components, which can increase the portability of use/utilization and storage.

According to a further aspect, the present invention also relates to a method for constructing a sgRNA sequencing library, which method uses the linker set as described above and comprises the following steps:
a) subjecting sgRNA to a linking reaction with the 3' linker under suitable conditions;
b) adding the 5' linker to the product obtained in the reaction of step a), and performing annealing and blocking under suitable conditions, such that the fourth non-random section of the 5' linker hybridizes with the first non-random section of the 3' linker to form a double strand;
c) subjecting the product obtained in step b) to a linking reaction under suitable conditions, such that the 5' linker is linked to the sgRNA;
d) subjecting the product obtained in step c) to a reverse transcription reaction under suitable conditions to obtain cDNA;
e) adding first and second sequencing linker primers with indexes to both ends of the cDNA and enriching the library.

In some embodiments, the linking reaction condition of linking the 3' linker comprises reacting at 22°C to 28°C for at least 1 h. In some embodiments, the linking reaction condition of linking the 3' linker comprises incubating at 16°C for 3 h. In some embodiments, the linking reaction condition of linking the 3' linker comprises incubating at 16°C for 18 h.

In some embodiments, the linking reaction condition of linking the 5' linker comprises: incubating at 34°C to 40°C for at least 15 minutes, or incubating at 23°C to 28°C for at least 1 h, or incubating at 14°C to 18°C for at least 16 h.

The effect of blocking is to better link the UMA5 linker, reduce the generation of an RNA co-fold structure that affects linking efficiency, and reduce the dimer contamination of the linker, thereby improving the final library yield.

In some embodiments, the reaction conditions of the annealing and blocking comprise: incubating at 70°C to 80°C for at least 10 minutes, slow cooling to 20°C to 30°C at a rate of 0.3°C/s to 1°C/s (e.g., 0.5°C/s or 0.7°C/s), and incubating for at least 15 minutes.

In the present invention, preferably, the sgRNA comprises a phosphate modification at the 5'-end. In some embodiments, if the sgRNA does not comprise a phosphate modification at the 5'-end, step a) further comprises phosphorylating the 5'-end of the product obtained from the linking of the 3' linker.

In some embodiments, the enzyme used for the linking in step a) is selected from at least one of T4 RNA ligase 2, T4 RNA ligase 2, truncated, and T4 RNA ligase 2, truncated KQ.

In some embodiments, the linking reaction in step a) is performed in a buffer system comprising 7 mM to 13 mM Mg²⁺ and 0.7 mM to 1.3 mM DTT.

In some embodiments, the buffer system of the linking reaction in step a) further comprises PEG8000 at a concentration of preferably 10% to 30% (w/v), more preferably 12% to 25% (w/v), e.g., 13%, 14%, 15%, 16%, 17%, 18%, 19%, 20%, 21%, 22%, 23% or 24% (w/v). In the present invention, the "w/v" refers to g/100 mL.

In some embodiment, the enzyme used for the linking in step c) is T4 RNA ligase 1.

In some embodiments, the reaction in step c) is performed in a buffer system comprising 7 mM to 13 mM Mg²⁺ and 0.7 mM to 1.3 mM DTT.

In some embodiments, a structure that can be cleaved by a protease is comprised between the first loop-forming section and the second loop-forming section of the loop-forming DNA section of the 3' linker, and step d) further comprises a fragmentation reaction of using a protease to cleave the loop-forming DNA section. The protease is preferably as defined above, and is, for example, User enzyme.

In some embodiments, the sgRNA is 20 to 200 nt, e.g., 25 nt, 30 nt, 35 nt, 40 nt, 50 nt, 60 nt, 70 nt, 80 nt, 90 nt, 100 nt, 120 nt, 140 nt, 160 nt, 180 nt, or 200 nt in length.

The present invention further relates to a sgRNA sequencing method, which comprises:
1) constructing a sgRNA sequencing library using the method as described above;
2) sequencing the sgRNA sequencing library obtained in step 1); wherein preferably sequencing is performed using an Illumina sequencing platform.

In some embodiments, a sgRNA library is constructed using the method as described above. In some embodiments, a library of sgRNAs with a modification, such as 2'-O-methyl modification, phosphorothioate modification, or 2'-O-methyl 3' phosphorothioate modification, is constructed using the method as described above.

The present invention further relates to the use of the 3' linker as described above or the linker set as described above in the construction of a sgRNA library.

Subjects for sequencing can be human, animals (such as rats, mice, cats, dogs, horses, cows, sheep, pigs, chickens, ducks, geese, quails, pigeons, nematodes, zebrafish), plants (such as rice, *Arabidopsis thaliana,* wheat, corn), and microorganisms (various viruses, bacteria, or fungi). It is readily understood that the above methods are universal methods that can be applied to different species and for different purposes.

The embodiments of the present invention will be described in detail below with reference to the examples. It should be understood that these examples are merely intended to illustrate the present invention but not to limit the scope of the present invention. The experimental methods in the following examples, where no specific conditions are specified, can be performed according to the instructions provided in the present invention, experimental manual or conventional conditions in the art, other experimental methods known in the art, or the conditions suggested by the manufacturer.

In the following specific examples, the measurement parameters related to the components of the raw materials may have slight deviations within the weighing accuracy, unless otherwise specified. When temperature and time parameters are involved, acceptable deviations due to instrument testing accuracy or operating accuracy are allowed.

### Example 1: sgRNA library construction and sequencing using UMA3 and UMAS linkers

Sample source: artificially synthesized sgRNA standard, wherein the sequence was 100 nt in length and has chemical modifications at the 5'-end and the 3'-end.

The example was performed according to a method for constructing a sgRNA next generation sequencing library.

The sources of the used reagents, etc. are as shown in the table below:

| **Name** | **Catalog No.** | **Manufacturer** |
|---|---|---|
| T4 RNA ligase 2, truncated KQ | M0373L | NEB |
| PEG 8000 | M0373L | NEB |
| T4 RNA ligase reaction buffer (10×) | M0373L | NEB |
| T4 RNA ligase 1 | M0204S | NEB |
| T4 RNA ligase reaction buffer (10×) | M0204S | NEB |
| ATP, 10 mM | M0204S | NEB |
| T4 phosphokinase | M0201S | NEB |
| T4 phosphokinase reaction buffer | M0201S | NEB |
| M-MuLV reverse transcriptase & 50000U/EA | M0253L | NEB |
| 10× M-MuLV buffer | M0253L | NEB |
| 10 mM dNTP Mix | 10124ES80 | Yeasen Biotechnology (Shanghai) Co., Ltd. |

### 1. Sample denaturation

1 µg -10 µg of sgRNA sample was taken, with a total volume of 6.5 µL. If the volume was less than 6.5 µL, the volume was made up to 6.5 µL with nuclease-free water.

Reaction conditions: reaction at 70°C for 2 min to form a single strand, and immediate placement on ice for at least 1 min.

### 2. Linking of 3' linker

A UMA3 linker was linked. The linker sequence was:
GTATCGTNNNNNNAGATCGGAAGAGCGTCGTGTAGGGAAAGAGTGTdUGTGACTGGAGTTCAGACGT GTGCTCTTCCGATCT (SEQ ID NO: 10). The linker sequence had an APP modification at the 5'-end and an NH₂ modification at the 3'-end. The reaction steps were as follows:

| | |
|---|---|
| Product in the previous step | 6.5 µL |
| UMA3 linker (10 µM) | 1 µL |
| 3' linking reaction buffer, comprising: | 5.5 µL |
| 136 mM Tris-HCl (pH 7.5) | |
| 27 mM MgCl₂ | |
| 2.7 mM DTT | |

| | |
|---|---|
| 36% W/V polyethylene glycol 8,000 MW | |
| 3' linking reaction enzyme mix, comprising: | 2 µL |
| T4 RNA ligase 2 (200,000 units/mL) | |
| Murine ribonuclease inhibitor (40,000 units/mL) | |
| Total volume | 15 µL |

Reaction conditions: reaction at 25°C for 1 h, and immediate placement on ice.

### 3. Phosphorylation

| | |
|---|---|
| Product in the previous step | 15 µL |
| Phosphorylation reaction buffer, comprising: | 4 µL |
| 350 mM Tris-HCl (pH 7.6) | |
| 50 mM MgCl₂ | |
| 25 mM DTT | |
| 5 mM ATP | |
| Phosphorylation reaction enzyme mix, comprising: | 2 µL |
| T4 phosphokinase (10,000 units/mL) | |
| Murine ribonuclease inhibitor (40,000 units/mL) | |
| Total volume | 21 µL |

Reaction conditions: reaction at 37°C for 30 min, reaction at 65°C for 20 min, and immediate placement on ice.

### 4. Blocking of 5' linker

The UMA5 linker sequence was:
ACACUCUUUCCCUACACGACGCUCUUCCGAUCUNNNNNNACGAUAC (SEQ ID NO: 11). 1 µL of the linker was taken and added into a new PCR tube, the tube was placed on a PCR instrument and incubated at 70°C for 2 min, and then immediately placed on ice; the denatured linker must be used up within 30 min.

| | |
|---|---|
| Product in the previous step | 21 µL |
| UMA5 linker (20 µM) | 1 µL |
| Total volume | 22 µL |

Reaction conditions: reaction at 75°C for 10 min, cooling to 25°C at 0.5°C/s, reaction at 25°C for 15 min, and immediate placement on ice.

### 5. Linking of 5' linker

| | |
|---|---|
| Product in the previous step | 22 µL |
| 5' linking reaction buffer, comprising: | 6 µL |
| 250 mM Tris-HCl (pH 7.5) | |
| 50 mM MgCl₂ | |
| 5 mM DTT | |
| 5 mM ATP | |
| 5' linking reaction enzyme mix, comprising: | 3 µL |
| T4 RNA ligase 1 (10,000 units/ml) | |
| Murine ribonuclease inhibitor (40,000 units/ml) | |
| Total volume | 31 µL |

Reaction conditions: reaction at 25°C for 1 h, and immediate placement on ice.

### 6. Reverse transcription

| | |
|---|---|
| Product in the previous step | 31 µL |
| Reverse transcription reaction buffer, comprising: | 7 µL |
| 285 mM Tris-HCl (pH 8.3) | |
| 428 mM KCl | |
| 17 mM MgCl₂ | |
| 57 mM DTT | |
| 4.3 mM dNTP Mix | |
| Reverse transcription reaction enzyme mix, comprising: | 2 µL |
| M-MuLV reverse transcriptase (200,000 units/ml) | |
| Murine ribonuclease inhibitor (40,000 units/ml) | |
| Total volume | 40 µL |

Reaction conditions: reaction at 42°C for 60 min, reaction at 85°C for 15 min, and immediate placement on ice.

### 7. PCR amplification

Indexes were linked to both ends of the product in the previous step by PCR, and a library was constructed. The 5'-end second sequencing linker primer sequence was:
AATGATACGGCGACCACCGAGATCTACACCGTCCGTGCACACTCTTTCCCTACACGAC (SEQ ID NO: 5), and the 3'-end first sequencing linker primer sequence was:
CAAGCAGAAGACGGCATACGAGATCATGCCATGTGACTGGAGTTCAGACGTGT (SEQ ID NO: 6).

| | |
|---|---|
| Product in the previous step | 40 µL |
| PCR amplification reaction buffer: | 50 µL |
| KAPA HiFi Hotstart ReadyMix | |

| | |
|---|---|
| PCR amplification reaction primers, comprising: | 10 µL |
| UMA P1 (10 µM) | |
| UMA P2 (10 µM) | |
| Total volume | 100 µL |

The reaction conditions were as follows:
step 1: reaction at 98°C for 45 s
step 2: reaction at 98°C for 15 s
step 3: reaction at 60°C for 30 s
step 4: reaction at 72°C for 30 s
step 5: reaction at 72°C for 1 min
step 6: hold at 4°C
with 12 to 30 cycles set in step 2 to step 4 according to the starting amount (1-10 µg) of the sample.

### 8. Library purification

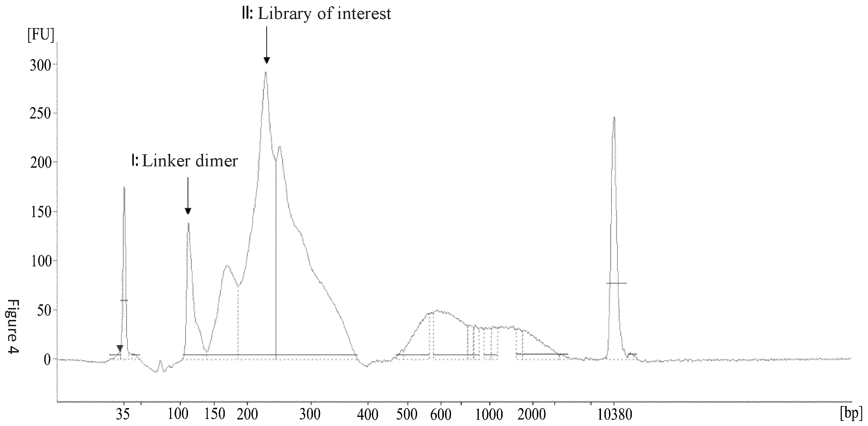

First, 0.8× Ampure XP magnetic beads were added to combine with the PCR products, then 0.2× Ampure XP magnetic beads were added to combine with the supernatant, and the products were redissolved in nuclease-free water or Elution Buffer. The recovered products were detected using an Agilent 2100 high sensitivity DNA chip. The results are as shown in the table below and FIG. 4. The PCR products of the sample library were concentrated at about 200 to 300 bp, with the target fragments being relatively concentrated.

| Distribution of fragments (bp) | Proportion (%) |
|---|---|
| 100-162 | 9% |
| 163-200 | 11% |
| 201-300 | 52% |
| 301-1000 | 21% |

It can be seen from the above results that the method for constructing the sgRNA second generation sequencing library of the present invention can successfully implement sgRNA library construction and sequencing.

### Example 2: sgRNA library construction and sequencing using commercially available linker for small RNA library construction

A small RNA library construction kit was purchased from NEB, and the sample used was the same as that in Example 1. After operation steps, such as sample denaturation, linking of 3' linker, blocking of 5' linker, linking of 5' linker, reverse transcription, PCR amplification and library purification, the final results were obtained. The results are as shown in Table 5, and it can be seen that the proportion of the target sequences of 200-300 bp is 0%, and therefore the commercially available linker sequence cannot construct a library of sgRNAs with a modification.

### Example 3: sgRNA library construction and sequencing using UMA3 and UMAS linkers

The library construction method and related reagents used were as described in Example 1. The UMA3 linker sequence was:
GTATCGTNNNNNNAGATCGGAAGAGCACACGTCTGAACTCCAGTCACdUACACTCTTTCCCTACACGACGCT CTTCCGATCT (SEQ ID NO: 12). The linker had an APP modification at the 5'-end and an NH₂ modification at the 3'-end. The UMA5 linker sequence was:
ACACUCUUUCCCUACACGACGCUCUUCCGAUCUNNNNNNACGAUAC (SEQ ID NO: 11).

For PCR amplification, the 5'-end second sequencing linker primer sequence was:
AATGATACGGCGACCACCGAGATCTACACCGTCCGTGCACACTCTTTCCCTACACGAC (SEQ ID NO: 5), and the 3'-end first sequencing linker primer sequence was:
CAAGCAGAAGACGGCATACGAGATCACTGACCTCAAGTCTGCACACGAGAAGGCTAGA (SEQ ID NO: 13).

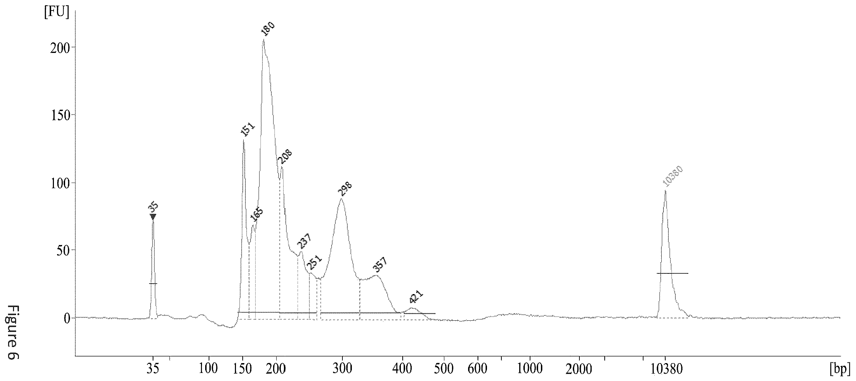

After library purification, the final results were obtained. The results are as shown in the table below and FIG. 6.

| Distribution of fragments (bp) | Proportion (%) |
|---|---|
| 100-162 | 8% |
| 163-200 | 37% |
| 201-300 | 38% |
| 301-1000 | 18% |

### Example 4: sgRNA library construction and sequencing using linear linker for small RNA library construction

A commercially available linear linker for small RNA library construction was used for library construction, and the ligase, reverse transcriptase and reagents used were the same as those in Example 1. The sample used was the same as that in Example 1. After operation steps, such as sample denaturation, linking of 3' linker, phosphorylation, blocking of 5' linker, linking of 5' linker, reverse transcription, PCR amplification and library purification, the final results were obtained. The results are as shown in Table 7, and it can be seen that the proportion of the target sequences of 200-300 bp is relatively low, while the proportion of linker dimers is very high.

The above examples merely represent several embodiments of the present invention, giving specifics and details thereof, but should not be understood as limiting the scope of the present patent of invention thereby. It should be noted that those of ordinary skill in the art could also make several alterations and improvements without departing from the spirit of the present invention and these would all fall within the scope of protection of the present invention. Therefore, the scope of protection of the present patent of invention shall be in accordance with the appended claims, and the description and accompanying drawings can be used to explain the content of the claims.

## Claims

1. A sgRNA sequencing 3' linker, sequentially comprising the following sections from a 5'-end to a 3'-end: a first non-random section, a first random section, a second non-random section, a loop-forming DNA section, and a third non-random section,
wherein the first non-random section is used for being linked to the 3'-end of the sgRNA;
the first random section comprises 3, 4, 5, 6, 7, 8, 9, 10, 11 or 12 basic groups;
the second non-random section is reversely complementary to the third non-random section so as to form a neck ring structure in conjuncture with the loop-forming DNA section; the third non-random section is used as a primer for sgRNA reverse transcription and replication;
the loop-forming DNA section is composed of a first loop-forming section and a second loop-forming section from the 5'-end to the 3'-end;
the third non-random section and the second loop-forming section can be combined with the first sequencing linker primer sequence in a complementary pairing mode.

2. The 3' linker according to claim 1, wherein the first non-random section comprises 5, 6, 7, 8, 9, 10, 11 or 12 basic groups.

3. The 3' linker according to claim 1, wherein the third non-random section comprises 2 to 31 basic groups, preferably comprises a sequence set forth in SEQ ID NO: 1.

4. The 3' linker according to claim 3, wherein the second loop-forming section comprises 3 to 33 basic groups, preferably comprises 10 to 30 basic groups, and more preferably comprises a sequence set forth in SEQ ID NO: 3.

5. The 3' linker according to claim 4, wherein the third non-random section and the second loop-forming section comprise a total of 34 basic groups.

6. The 3' linker according to claim 1, wherein the sequence of the first loop-forming section comprises a sequence set forth in SEQ ID NO: 2.

7. The 3' linker according to any one of claims 1 to 6, wherein a structure that can be cleaved by a protease is comprised between the first loop-forming section and the second loop-forming section, and the structure is preferably one or more dUs.

8. The 3' linker according to any one of claims 1 to 6, comprising a nucleotide modification at the 5'-end and/or 3'-end thereof; preferably, the 3' linker comprises an adenylation modification at the 5'-end, and preferably, the 3' linker comprises an amino modification at the 3'-end.

9. A linker set, comprising the 3' linker according to any one of claims 1 to 8 and a 5' linker used for being linked to the 5'-end of the sgRNA,
wherein the 5' linker is composed of ribonucleotides, and sequentially comprises a second sequencing linker primer-binding section, a second random section, and a fourth non-random section from a 5'-end to a 3'-end; the second random section comprises 3, 4, 5, 6, 7, 8, 9, 10, 11 or 12 basic groups; the fourth non-random section is reversely complementary to the first non-random section.

10. The linker set according to claim 9, wherein the second sequencing linker primer-binding section comprises 17 to 33 basic groups; preferably, the second sequencing linker primer-binding section comprises a sequence set forth in SEQ ID NO: 4.

11. A kit, comprising the linker set according to claim 9 or 10.

12. The kit according to claim 11, further comprising at least one of the following components:
·an RNA ligase, preferably comprising:
i) T4 RNA ligase 1, and/or
ii) at least one of T4 RNA ligase 2, T4 RNA ligase 2, truncated, and T4 RNA ligase 2, truncated KQ;
·a linking buffer suitable for the RNA ligase, preferably a buffer system comprising 7 mM to 13 mM Mg²⁺ and 0.7 mM to 1.3 mM DTT;
·an enzyme or an enzyme composition having uracil-DNA glycosylase activity and AP-endonuclease activity; preferably a mixture of uracil-DNA glycosylase and Endo VIII, and more preferably User enzyme;
·a reverse transcriptase;
·a reverse transcription reaction buffer;
·a DNA polymerase;
·a PCR amplification buffer for cDNA amplification;
·a T4 phosphokinase;
·a T4 phosphokinase reaction buffer;
·a first sequencing linker primer for being combined with the third non-random section and the second loop-forming section in a complementary pairing mode, and a second sequencing linker primer for being combined with the second sequencing linker primer-binding section in a complementary pairing mode;
·dNTPs;
·water.

13. A method for constructing a sgRNA sequencing library, wherein the method uses the linker set according to claim 7 or 8 and comprises the following steps:
a) subjecting sgRNA to a linking reaction with the 3' linker under suitable conditions;
b) adding the 5' linker to the product obtained in the reaction of step a), and performing annealing and blocking under suitable conditions, such that the fourth non-random section of the 5' linker hybridizes with the first non-random section of the 3' linker to form a double strand;
c) subjecting the product obtained in step b) to a linking reaction under suitable conditions, such that the 5' linker is linked to the sgRNA;
d) subjecting the product obtained in step c) to a reverse transcription reaction under suitable conditions to obtain cDNA;
e) adding first and second sequencing linker primers with indexes to both ends of the cDNA and enriching the library.

14. The method according to claim 13, wherein the reaction conditions of the annealing and blocking in step b) comprise: incubating at 70°C to 80°C for at least 10 minutes, slow cooling to 20°C to 30°C at a rate of 0.3°C/s to 1°C/s, and incubating for at least 15 minutes.

15. The method according to claim 13, wherein step a) further comprises phosphorylating the 5'-end of the product obtained from the linking of the 3' linker.

16. The method according to any one of claims 13 to 15, wherein the enzyme used for the linking reaction in step a) is selected from at least one of T4 RNA ligase 2, T4 RNA ligase 2, truncated, and T4 RNA ligase 2, truncated KQ.

17. The method according to claim 16, wherein the linking reaction in step a) is performed in a buffer system comprising 7 mM-13 mM Mg²⁺ and 0.7 mM-1.3 mM DTT.

18. The method according to claim 17, wherein the buffer system of the linking reaction in step a) further comprises PEG8000 at a concentration of 10% to 30% (w/v), preferably 12% to 25% (w/v).

19. The method according to any one of claims 13 to 15, 17 and 18, wherein the enzyme used for the linking reaction in step c) is T4 RNA ligase 1.

20. The method according to claim 19, wherein the linking reaction in step c) is performed in a buffer system comprising 7 mM-13 mM Mg²⁺ and 0.7 mM-1.3 mM DTT.

21. The method according to any one of claims 13 to 15, 17, 18 and 20, wherein a structure that can be cleaved by a protease is comprised between the first loop-forming section and the second loop-forming section of the loop-forming DNA section of the 3' linker, step d) further comprises a fragmentation reaction of using a protease to cleave the loop-forming DNA section, and the protease is preferably User enzyme.

22. The method according to any one of 13 to 15, 17, 18 and 20, wherein the sgRNA is 20 to 200 nt in length.

23. A sgRNA sequencing method, comprising:
1) constructing a sgRNA sequencing library using the method according to any one of claims 11 to 22;
2) sequencing the sgRNA sequencing library obtained in step 1); wherein preferably sequencing is performed using an Illumina sequencing platform.

24. Use of the 3' linker according to any one of claims 1 to 8 or the linker set according to claim 9 or 10 in the construction of a sgRNA library.

25. A sgRNA sequencing library constructed by the method according to any one of claims 13 to 15, 17 and 18.
